# EUROPÄISCHE PATENTSCHRIFT

(11) **EP 0 589 208 B1**
(45) Veröffentlichungstag und Bekanntmachung des Hinweises auf die Patenterteilung: **17.05.1995**
(21) Anmeldenummer: 93113313.6
(22) Anmeldetag: 20.08.1993
(51) Int. Cl.: C07D 251/28

(54) **Verfahren zur Herstellung von Suspensionen von Cyanurchlorid in wässrigen Flüssigkeiten**
Process for the preparation of suspensions of cyanuric chloride in aqueous fluids
Procédé pour la préparation de suspensions de chlorure de cyanuryle dans des fluides aqueuses

(30) Priorität: 25.09.1992 DE 4232117
(43) Veröffentlichungstag der Anmeldung: 30.03.1994
(73) Patentinhaber: DEGUSSA AG, 60311 Frankfurt (DE)
(72) Erfinder: Vanheertum, Rudolf Dr., B-2180 Ekeren-Antwerpern (BE); Hendricx, Richard, B-2170 Merksem-Antwerpen (BE); Kriebitzsch, Norbert Dr., B-2950 Kapellen (BE); Van de Velde, Francois, B-2170 Merksem-Antwerpen (BE); Schirrmacher, Rüdiger, D-63454 Hanau (DE)

(56) Entgegenhaltungen:
- EP-A- 0 094 665
- EP-A- 0 200 151
- DE-C- 2 850 242

## Beschreibung

Die Erfindung betrifft ein Verfahren zur Herstellung von Suspensionen von Cyanurchlorid in wäßrigen Flüssigkeiten, insbesondere in Wasser.

Cyanurchlorid ist ein wichtiges Ausgangsprodukt zur Herstellung von Herbiziden, optischen Aufhellern, Reaktivfarbstoffen sowie Kunststoff- und Kautschukadditiven. Die Herstellung dieser Stoffe geschieht durch Substitution der Chloratome des Cyanurchlorids durch andere Liganden, beispielsweise solche mit Amin-, Mercapto- oder Hydroxyfunktionen. Die Umsetzungen werden teils in organischen Lösungsmitteln, zu einem großen Teil aber mit Suspensionen von feinteiligem Cyanurchlorid in Wasser oder einem wäßrig-organischen Reaktionsmedium durchgeführt.

Die wäßrigen Cyanurchlorid-Suspensionen lassen sich durch Anmaischen des feinteiligen Cyanurchlorid-Pulvers, wie es im Handel erhältlich ist, in kaltem Wasser, mit oder ohne Zusatz von Netzmitteln, erhalten. Tiefe Temperaturen verlangsamen die unerwünschte Hydrolysereaktion des Cyanurchlorids. Nachteilig an dieser Methode zur Suspensionsherstellung ist der aufwendige Umgang mit Schüttgütern mit Gebindegrößen zwischen 50 und 1000 kg. Erschwert wird der Umgang durch die physiologischen und korrosiven Eigenschaften des Cyanurchlorids.

In der Vergangenheit sind daher Versuche unternommen worden, Cyanurchlorid in Form seiner pumpbaren Schmelze zur Herstellung wäßriger Suspensionen ebenso einzusetzen, wie es bei Verwendung gewisser organischer Lösungsmittel praktiziert werden kann. Hierzu müssen die Erstarrungsenthalpie und die spezifische Wärme zwischen der Schmelzetemperatur und Temperaturen um vorzugsweise nahe 0 ^{o}C abgeführt werden. Dabei darf die entstehende Suspension zwischenzeitlich keine Temperaturen erreichen, bei denen die Hydrolysereaktion zu stark wird, außerdem müssen die Cyanurchlorid-Partikel möglichst feinteilig sein, um die anschließende Synthese rasch und selektiv verlaufen zu lassen.

Aus der DE-PS 16 70 731 und DE-OS 21 62 064 sind Verfahren bekannt, wonach geschmolzenes Cyanurchlorid im Dampfraum eines Rührreaktors, der teilweise mit Wasser gefüllt ist, versprüht wird. Ein Versprühen von Cyanurchloridschmelze in einen Gasraum führt aber dazu, daß aus der Schmelze verdampfendes Cyanurchlorid im Gasraum desublimiert und an der nicht mit Wasser benetzten Wandung des Sprühreaktors Krusten bildet, die zu Verstopfungen im Apparat und zu Brocken in der Suspension führen.

In der DE-PS 28 50 242 sowie DE-PS 28 50 308 wird ein sich nach unten brustförmig zu einer Ausflußöffnung verjüngender Reaktor mit im Reaktorkopf angeordneter Sprühdüse beschrieben. In dem Reaktor wird ein rotierender Flüssigkeitsring erzeugt, auf dessen Oberfläche die Cyanurchloridschmelze verdüst wird. Um Anbackungen zu vermeiden, wird die Rotationsgeschwindigkeit des Flüssigkeitsringes so hoch gewählt, daß der Ring sich am Deckel des Reaktors nahezu vollständig um die Sprühdüse schließt. Dies läßt sich regeltechnisch im rauhen Dauerbetrieb nur schwierig jederzeit gewährleisten, außerdem bietet diese Ausführungsart nur eine graduelle Verbesserung und läßt, da der Flüssigkeitsring sich auf einer entsprechend engen Ausflußöffnung abstützen muß, nur verhältnismäßig niedrig konzentrierte Suspensionen zu.

In der EP-B 0 094 665 wird ein Verfahren zur Herstellung einer Suspension von Cyanurchlorid in Wasser oder zur Umsetzung von Cyanurchlorid in Ammoniak oder Aminen beschrieben, wobei die Nachteile der vorbekannten Verfahren möglichst weitgehend vermieden werden sollten. Das Versprühen von geschmolzenem Cyanurchlorid in Wasser oder eine wäßrige Ammoniak- oder Aminlösung oder -Suspension erfolgt hierbei mittels einer Sprühdüse, welche in die Flüssigkeit eintaucht. Vor dem Starten des Einsprühvorgangs wird aber der Flüssigkeitspegel im Umgebungsbereich der Düse soweit abgesenkt, daß die Düsenspitze nicht mehr in die Flüssigkeit eintaucht, und erst nach dem Starten wieder angehoben, so daß die Düse wieder eintaucht. Die bereits vorerwähnten Probleme, welche für das Versprühen von Cyanurchloridschmelze in einen Gasraum charakteristisch sind, werden gerade in der kritischen Phase des An- und Abfahrens des Sprühprozesses bei diesem Verfahren ebenfalls nicht behoben.

Alle erwähnten Verfahren vermeiden es, daß das Einleitorgan, also insbesondere die Düse und deren Zuführungsleitung für geschmolzenes Cyanurchlorid, beim An- und Abfahren innen mit Wasser benetzt werden, da sich andernfalls aus zurückgebliebenen Cyanurchloridresten die Düse verstopfende und die Cyanurchloridsuspension verunreinigende Hydrolyseprodukte bilden würden.

Aufgabe der vorliegenden Erfindung ist es, ein Verfahren zur Verfügung zu stellen, das die Nachteile der vorbekannten Verfahren beim An- und Abfahren vermeidet und die Herstellung hochreaktiver wäßriger Cyanurchloridsuspensionen gestattet.

Gefunden wurde ein Verfahren zur Herstellung einer Suspension von Cyanurchlorid in einer wäßrigen Flüssigkeit, insbesondere in Wasser, wobei die wäßrige Flüssigkeit außer Wasser darin gelöste organische Lösungsmittel und/oder Reaktionspartner zur weiteren Umsetzung des gebildeten suspendierten Cyanurchlorids enthalten kann, durch Versprühen von geschmolzenem Cyanurchlorid in die genannte, gegebenenfalls bereits suspendiertes Cyanurchlorid enthaltende wäßrige Flüssigkeit unter Verwendung einer Sprühvorrichtung mit einer in die wäßrige Flüssigkeit eingetauchten Sprühdüse und Aufrechterhaltung einer Temperatur von 0 bis 50 ^{o}C in der Suspension, das dadurch gekennzeichnet ist, daß die Sprühdüse auch in der Anfahr- und Abstellungsphase stets in die wäßrige Flüssigkeit eingetaucht ist und man unmittelbar vor und nach dem Versprühen durch die mit geschmolzenem Cyanurchlorid in Berührung kommenden Teile der Sprühvorrichtung überhitzten Wasserdampf leitet.

Die weiteren Ansprüche richten sich auf bevorzugte Ausführungsformen des Verfahrens, insbesondere solche, wobei besonders hochreaktive Cyanurchloridsuspensionen zugänglich sind.

Wesentliches Kennzeichen der Erfindung ist, daß durch die Düsenkonstruktion, welche stets abgetaucht ist, bei der Inbetriebnahme und bei Abstellungen überhitzter Wasserdampf geleitet wird. Der überhitzte Wasserdampf weist eine Temperatur im Bereich von 150 bis 300 ^{o}C, insbesondere 200 bis 290 ^{o}C, auf.

Es wurde nämlich gefunden, daß Cyanurchlorid mit überhitztem Wasserdampf keine Hydrolysereaktion in störendem Ausmaß eingeht, obwohl es mit flüssigem Wasser bei höheren Temperaturen sehr schnell und unter Umständen heftig reagiert. Diese Erkenntnis wird ausgenutzt, indem man das eventuell in der Düse beziehungsweise in dem Einleitrohr stehende Wasser vor dem Anfahren durch trockenen Wasserdampf verdrängt und unmittelbar anschließend die Cyanurchlorid-Schmelze durchströmen läßt; bei Abstellung verfährt man in umgekehrter Reihenfolge und befreit dadurch die Einleitorgane von restlichem Cyanurchlorid. Das Einleiten des Dampfes unterstützt gleichzeitig die Wirkung der nach den Regeln der Technik am Einleitorgan angebrachten Begleitheizung und Isolierung in den kritischen Phasen des An- bzw. Abstellens.

Geeignete Vorrichtungen zur Durchführung des Verfahrens sind in den Zeichnungen 1 bis 4 dargestellt.

Fig. 1 zeigt den schematischen Aufbau einer Anlage zur Herstellung der Cyanurchloridsuspensionen.

Fig. 2 zeigt einen Längsschnitt durch eine dampfbeheizte Sprühdüse.

Fig. 3 zeigt einen Längsschnitt durch eine Sprühvorrichtung mit einer Vorrichtung zur Naßvermahlung unmittelbar nach der Suspensionsbildung.

Fig. 4 zeigt einen Querschnitt durch eine Sprühvorrichtung zum Versprühen eines Cyanurchloridschmelze/Dampf-Gemischs.

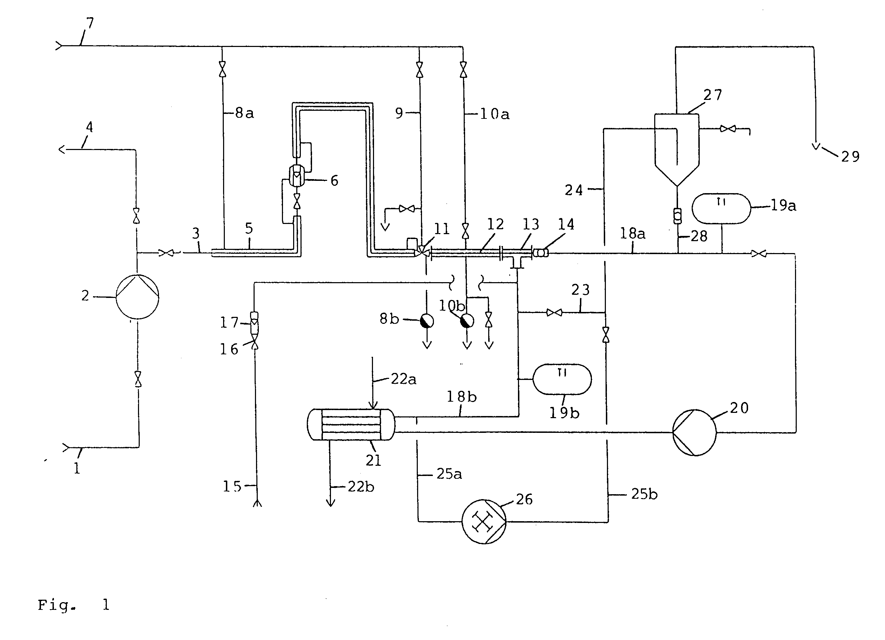

Figur 1 zeigt den schematischen Aufbau einer Anlage zur Durchführung des erfindungsgemäßen Verfahrens. Cyanurchloridschmelze wird aus einem (nicht gezeigten) Vorratsbehälter über Leitung 1, Pumpe 2 und Leitung 3 der Sprühanlage zugeführt; über Leitung 4 kann Schmelze rezykliert werden. Über die dampfbeheizte Leitung 5 mit einer Regulier- und Meßvorrichtung (Regelventil und Rotameter) 6 für den Volumenstrom gelangt die Schmelze zu dem Dreiwegeventil 11 und von dort in die eigentliche Sprühvorrichtung, welche die Sprühdüse 12 und die Kammer 13 zur Zuführung der wäßrigen Flüssigkeit und Suspensionsbildung umfaßt; das Schauglas 14 befindet sich im Bereich der Düsenspitze. Wasserdampf aus Leitung 7 wird über Leitung 8a zur Beheizung der Leitung 5, der Meß- und Reguliervorrichtung 6 und des Dreiwegeventils 11 zugeführt; das Kondensat wird über Kondensator 8b abgenommen. Mittels Leitung 10a wird Dampf dem Heizmantel der Sprühdüse zugeführt; Kondensat wird über den Kondensator 10b abgenommen. Dem Dreiwegeventil 11 wird über Leitung 9 Dampf zugeführt, um die Düse 12 vor dem Anfahren und nach dem Abfahren von Cyanurchlorid zu befreien. Die Leitungen 18a und 18b, Pumpe 20, Kühler 21 (22a und 22b stehen für die Zu- und Ableitung des Kühlmediums) und Kammer 13 bilden einen Suspensionskreilauf, dem über die mit einem Regelventil 16 und einer Meßvorrichtung (Rotameter) 17 ausgestattete Leitung 15 Wasser bzw. feststofffreie wäßrige Flüssigkeit zugeführt wird; 19a und 19b bedeuten Temperaturmeßstellen vor und nach dem Kühler. Über die Leitungen 23 und 24 wird Suspension aus dem genannten Suspensionskreislauf der Pumpenvorlage 27 zugeführt und über Leitung 28 in Leitung 18a zurückgeführt. Über Leitung 29 wird Suspension entnommen und der Verwendung zugeführt.

Bei Bedarf kann in den Suspensionskreislauf eine Naßvermahlung integriert sein, wobei der gesamte oder ein Teilstrom über die Naßmühle 26 und Leitungen 25a und 25b geführt wird. Sofern die Suspension konzentriert werden soll, kann dies mittels eines Hydrozykons erfolgen.

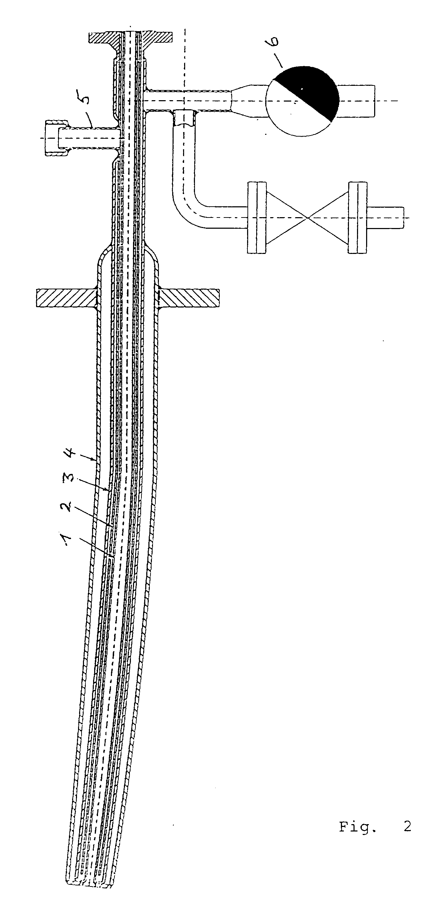

Die angestrebte Feinkörnigkeit und damit hohe Reaktionsfähigkeit des Cyanurchlorids in der Suspension läßt sich über die Ausströmgeschwindigkeit der Cyanurchloridschmelze beeinflussen. Je höher die Austrittsgeschwindigkeit der Schmelze am Düsenmund beziehungsweise je kleiner die Düsenöffnung ist, desto feinkörniger fällt das suspendierte Cyanurchlorid an. Zum Versprühen eignen sich übliche Einstoffsprühdüsen. Eine bevorzugte Einstoffdüse zeigt Fig. 2:
Im zentralen Rohr (1) der Düse strömt Cyanurchloridschmelze. Zur Beheizung wird im Mantelrohr (2) über Anschluß (5) Dampf zugegeben. Nach Umlenkung an der Düsenspitze strömen Dampf und Kondensat durch Mantelrohr (3) zum Kondensatablaß (6). Das Mantelrohr (4) ist ein Hohlraum für die thermische Isolierung der Düse.

Gemäß einer anderen bevorzugten Ausführungsform wird eine Sprühvorrichtung mit einer Zweistoffdüse verwendet, wobei dem in der Düse zentral geführten Cyanurchlorid-Schmelzstrahl mittels eines am Düsenmund angebrachten Ringspalts die den Schmelzstrahl umhüllende wäßrige Flüssigkeit zugeführt wird. Bei der wäßrigen Flüssigkeit kann es sich um die Suspension selbst, vorzugsweise aber uni dem System zugeführte feststofffreie wäßrige Flüssigkeit, insbesondere Frischwasser, handeln. Vordruck und Düsendurchmesser werden so aufeinander abgestimmt, daß die gewünschte Austrittsgeschwindigkeit resultiert.

Die zu versprühende Cyanurchloridschmelze weist im allgemeinen eine Temperatur zwischen 146 und 190 ^{o}C auf; sofern die Schmelze unter erhöhtem Druck vorliegt, kann diese auch bei einer Temperatur oberhalb 190 ^{o}C versprüht werden.

Unter der wäßrigen Flüssigkeit, in welcher die Suspension gebildet und gegebenenfalls gleich weiter umgesetzt wird, wird insbesondere Wasser verstanden. Zusätzlich können darin gelöste organische Lösungsmittel, wie beispielsweise Ketone, insbesondere höhere, mit Wasser azeotropbildende Ketone, enthalten sein. Die wäßrige Flüssigkeit kann ferner Reaktionskomponenten zur unmittelbaren Umsetzung der sich bei der Versprühung bildenden Cyanurchloridpartikel enthalten. Vorzugsweise handelt es sich bei den Reaktionskomponenten um Ammoniak oder Amine sowie gegebenenfalls basische Komponenten zur Neutralisation von bei der Umsetzung gebildeter Chlorwasserstoffsäure.

Mit dem erfindungsgemäßen Verfahren lassen sich wäßrige Cyanurchloridsuspensionen mit bis über 50 Gew.-%, vorzugsweise 20 bis 50 Gew.-%, Cyanurchlorid herstellen. Die Suspension ist praktisch frei von Hydrolyseprodukten. Die Reaktivität der gebildeten Suspension läßt sich in einfacher Wiese in einem Test - siehe Beispiel 2 - ermitteln; darauf aufbauend lassen sich die zum Erhalt einer bestimmten Reaktivität erforderlichen Betriebsparameter für den Sprühprozeß einstellen.

Es ist selbstverständlich notwendig, durch geeignete Vorrichtungen zur Kühlung der Suspension, etwa mittels innen- und/oder außenliegender Kühler, für eine ausreichende Wärmeabfuhr zu sorgen. Die Temperatur der Suspension wird während des Sprühprozesses im allgemeinen im Bereich von 0 bis 30 ^{o}C, vorzugsweise unter 20 ^{o}C, gehalten. Gemäß einer bevorzugten Ausführungsform des Verfahrens befindet sich die Sprühvorrichtung, umfassend eine Düse mit Zuleitungen für die Cyanurchloridschmelze und den trockenen Wasserdampf, in eingetauchter Position in einem mit der wäßrigen Flüssigkeit befüllten Sprühreaktor; der Inhalt des Reaktors wird über einen außenliegenden Intensivkühler umgewälzt; dem Kreislauf wird frische wäßrige Flüssigkeit in der Menge zugeführt, wie Cyanurchloridsuspension abgezogen und bei Bedarf über einen Hydrocyclon konzentriert wird.

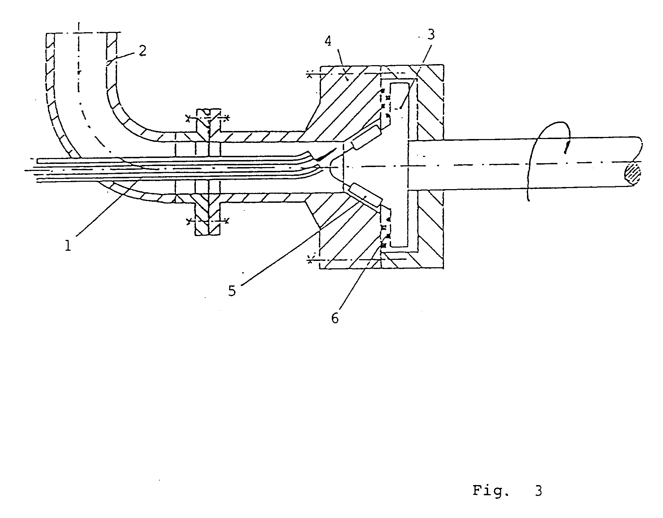

Bei Bedarf kann in den Suspensionskreislauf oder einen Seitenstrom desselben (wie in Fig. 1 dargestellt) auch eine Maschine zur Naßzerkleinerung, etwa eine Naßmühle, integriert werden. Vorzugsweise wird in diesem Fall, wie in Figur 3 dargestellt, die Sprühdüse unmittelbar vor die Saugseite einer solchen Naßmühle positioniert, da auf diese Weise nicht nur eine intensive Suspendierung, sondern gleichzeitig eine weitere Minimierung der Cyanurchlorid-Partikelgröße und damit Erhöhung der Reaktivität der Partikel erzielt wird. In Fig. 3 bedeuten: 1 eine lanzenartige dampfbeheizte Sprühdüse (Bauart gemäß Fig. 2) zur Zufuhr der Cyanurchloridschmelze, 2 Zuführungsleitung für die wäßrige Flüssigkeit, 3 Rotor der Naßmühle, 4 Stator der Naßmühle, 5 Laufkegel mit Messern, 6 Zone der Feinstzerkleinerung (Ablaufleitung aus der Mühle nicht gezeigt).

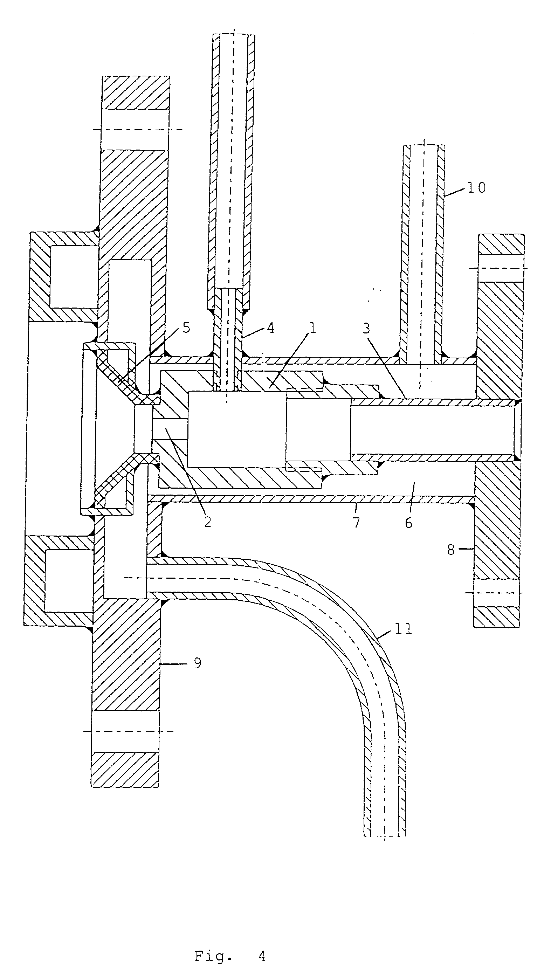

Es wurde ferner gefunden, daß besonders hochreaktive Cyanurchloridsuspensionen erhalten werden, wenn die Cyanurchloridschmelze nicht als solche, sondern im Gemisch mit überhitztem Wasserdampf versprüht wird. Das Gewichtsverhältnis Wasserdampf zu Cyanurchloridschmelze beträgt 1 zu größer 2 und liegt vorzugsweise zwischen 1 zu 15 bis 1 zu 25. Das Cyanurchlorid/Dampf-Gemisch wird zweckmäßigerweise unmittelbar vor dem Versprühen hergestellt. Derartige Gemische lassen sich in Zweistoffdüsen mit innenliegender Mischzone erzeugen und unmittelbar versprühen. Eine geeignete Düsenkonstruktion ist in Figur 4 dargestellt. In der dargestellten Zweistoffdüse wird der eigentliche Düsenkörper, bestehend aus der Mischkammer 1 mit der Düsenöffnung 2, dem Zuführungsrohr 3 für Cyanurchloridschmelze, dem Zuführungsrohr 4 für den beizumischenden Wasserdampf und dem Düsenmundstück 5, von einem mittels Dampf bezeihbaren Mantelraum 6 umgeben, der aus dem Rohr 7 und den Endstücken 8 und 9 gebildet wird; durch die Zuleitung 10 wird Dampf zum Beheizen zugeführt, durch Leitung 11 Kondensat abgeführt. Die unterschiedlichen Strömungsgeschwindigkeiten von Wasserdampf und Cyanurchloridschmelze in einer Düse erzeugen Druckwellen, die ein Aufreißen des Schmelzstromes in feinste schwebefähige Tropfen bewirken. Da der im Gemisch ausströmende Dampf in der wäßrigen Flüssigkeit kondensiert, werden Gasbelastungen vermieden, die bei Spülgasen - wie z. B. heißem Stickstoff - auftreten würden. Außerdem wird durch Wasserdampf kein dritter, unter Umständen störender Stoff eingebracht. Durch das Versprühen des Cyanurchloridschmelze/Dampf-Gemisches ist es möglich, hohe Ausströmungsgeschwindigkeiten bei geringem Vordruck und/oder breitem Düsenmund zu erzielen. Verstopfungen in der Düse werden auf diese Weise vollständig vermieden.

Die Vorteile der Erfindung liegen darin, daß die beim An- und Abfahren des Sprühprozesses bisher unvermeidlichen Probleme behoben werden konnten. Betriebsstörungen durch Verstopfungen der Sprühvorrichtung werden vermieden. Damit ist auch ein Sprühen kleiner Produktionschargen möglich. Außer durch die an sich bekannten Steuerungsmöglichkeiten der Teilchengröße und damit der Reaktivität der suspendierten Cyanurchloridpartikel durch Düsenvordruck, Düsenbauart und Düsendurchmesser kann die Reaktivität erfindungsgemäß weiter dadurch gesteigert werden, daß ein Cyanurchlorid/Dampf-Gemisch versprüht wird. Die erhöhte Reaktivität führt zu verkürzten Reaktionszeiten bei der sich anschließenden Umsetzung der Suspension mit Reaktionspartnern.

### Beispiel 1

Vor Versuchsbeginn werden die Leitungen und die Apparate, die mit der Cyanurchloridschmelze in Kontakt kommen, mit Wasserdampf (12 bar, 270 ^{o}C) vorgeheizt. In den Suspensionskreislauf - Apparatur gemäß Fig. 1 jedoch ohne Naßmühle - umfassend eine Einstoffsprühdüse (12) eine Vorrichtung (13) zur Umhüllung des Schmelzstrahls mit der wäßrigen Flüssigkeit, eine Pumpe (20), einen Kühler (21) und eine diese Aggregate verbindende Kreislaufleitung (18), wird Frischwasser vorgelegt; es werden 5 m³/Std. Wasser umgewälzt. Über das Dreiwegeventil (11) wird der Cyanurchloriddüse (12) Wasserdampf (12 bar, 270 ^{o}C) zugeführt. Nach 30 sec wird das Dreiwegeventil umgeschaltet. Hierdurch wird anstelle von Wasserdampf flüssiges Cyanurchlorid in die Düse geleitet. Die Frischwasserzufuhr (15) wird ebenfalls geöffnet. An der Austrittsöffnung der Düse, die in dem Wasserkreislauf abgetaucht ist, erscheint sofort eine feinteilige Suspension - erkennbar im Schauglas (14). Der Cyanurchloriddurchsatz wird auf 970 kg/Std., der Wasserdurchsatz auf 3.900 kg/Std. eingestellt. Die Cyanurchloridaustrittsgeschwindigkeit an der Düse (Öffnung 4 mm) beträgt somit 14,8 m/s. Die Temperatur wird auf 16 ^{o}C gehalten. Die Konzentration der aus dem Kreislauf gezogenen Suspension beträgt 20 Gew.-%. Mit Laserbeugung wurde eine durchschnittliche Korngröße von 29 »m bestimmt. Die Apparatur wird während 12 Minuten weiter betrieben. Beim Abfahren wird das Dreiwegeventil umgeschaltet und die Düse kurz mit Dampf gespült.

### Beispiel 2

In der im Beispiel 1 beschriebenen Apparatur werden 1.490 kg/Std. Frischwasser und 990 kg/Std. Cyanurchloridschmelze zugegeben. Für die Anfahrt wird die Cyanurchloriddüse während 30 sec mit 12 bar Dampf gespült, bevor die Cyanurchloridzugabe gestartet wird. Nach Beginn der Zugabe an Cyanurchloridschmelze wird die Temperatur der Suspension während der 12 minütigen Versuchsdauer auf 20 ^{o}C gehalten. Erhalten wird eine Suspension von 40 % Cyanurchlorid in Wasser; durchschnittliche Korngröße der Cyanurchloridpartikel 30 »m. Die Hydrolyse des suspendierten Cyanurchlorids an der Entnahmestelle (29) der Apparatur ist zu vernachlässigen. Beim Abfahren wird die Düse kurz mit Dampf gespült.

Die erhaltene 40 gew.-%ige Suspension von Cyanurchlorid in Wasser wird mittels einer bekannten Umsetzung mit Natriumhydrogensulfid zu Trismercapto-s-triazin auf ihre Reaktivität überprüft. Hierzu wird die Umsetzung einmal mit der erfindungsgemäß hergestellten Suspension und einmal als Blindversuch mit frisch suspendiertem pulverförmigen Cyanurchlorid mit einem mittleren Korndurchmesser von 33 »m durchgeführt.

In einem mit einem Rührer, einem Doppelmantel zur Kühlung, einem Thermometer und einem Zulauftrichter versehenen Laborreaktor von 1 Liter werden 281 g NaSH 37,8 % und 12 g NaOH 50 % vorgelegt. Unter Einhaltung einer Temperatur von 50 bis 60 ^{o}C werden 269 g 40 gew.-%ige Cyanurchloridsuspension und 88 g NaOH 50 % zugegeben. Nach einer Reaktionszeit von einer Stunde bei 50 ^{o}C wurde mittels Dünnschichtchromatographie in beiden Versuchen der quantitative Umsatz des Cyanurchlorids nachgewiesen.

Nach Zugabe von weiteren 54 g NaOH 50 % und 30 Min. Nachreaktion bei 50 ^{o}C wurde das Reaktionsgemisch bis auf 10 ^{o}C abgekühlt und das Trismercapto-s-triazintrinatriumsalz abfiltriert. Es wurden 253 g Filterkuchen erhalten. Das erhaltene Produkt war in beiden Fällen gut kristallin und ergibt in der HPLC-Analyse den gleichen Gehalt.

### Beispiel 3

Die Versuchsführung ist gegenüber Beispiel 2 dahingehend abgewandelt, daß als wäßrige Flüssigkeit 1.500 kg/Std. einer 10 gew.-%igen Lösung von Aceton in Wasser zusammen mit 990 kg/Std. Cyanurchloridschmelze durchgesetzt wird. Die erhaltene 40 gew.-%ige Suspension weist eine durchschnittliche Korngröße von 28 »m auf.

### Beispiel 4

Für diesen Versuch wird die in Beispiel 1 beschriebene Apparatur anstelle mit der dort verwendeten Einstoffdüse mit der in Figur 4 gezeigten Zweistoffdüse ausgerüstet. Die Cyanurchloridleitung und die Düse werden mit Wasserdampf (12 bar, 270 ^{o}C) vorgeheizt. Der Suspensionskreislauf wird mit Frischwasser befüllt und umgewälzt (5 m³/Std.). Die Austrittsöffnung der Düse beträgt 4,2 mm. Über einen Durchflußmesser wird der Zweistoffdüse über den Anschluß für Wasserdampf ((3) in Fig. 3) Wasserdampf (12 bar, 270 ^{o}C) in einer Menge von 16 kg/Std. zugeführt. Anschließend werden 344 kg/Std. Cyanurchloridschmelze in die Zweistoffdüse eingeleitet. Es werden 2.060 kg/Std. Frischwasser dem Kreislauf zugegeben und 2420 kg/Std. Suspension entnommen. Wasserdampf und Cyanurchloridschmelze werden während des Versuches kontinuierlich in die Zweistoffdüse eingespeist. Der Wasserdampf kondensiert sofort an der Austrittsstelle, die in der Kreislaufflüssigkeit abgetaucht ist. Die Konzentration der erhaltenen Suspension betrug 14 %, die durchschnittliche Korngröße 30 »m. Der Versuch wird nach 8 Min. beendet, indem erst die Cyanurchlorid- und anschließend die Dampfzufuhr unterbrochen werden.

## Patentansprüche

1. Verfahren zur Herstellung einer Suspension von Cyanurchlorid in einer wäßrigen Flüssigkeit, insbesondere in Wasser, wobei die wäßrige Flüssigkeit außer Wasser darin gelöste organische Lösungsmittel und/oder Reaktionspartner zur weiteren Umsetzung des gebildeten suspendierten Cyanurchlorids enthalten kann, durch Versprühen von geschmolzenem Cyanurchlorid in die genannte, gegebenenfalls bereits suspendiertes Cyanurchlorid enthaltende wäßrige Flüssigkeit unter Verwendung einer Sprühvorrichtung mit einer in die wäßrige Flüssigkeit eingetauchten Sprühdüse und Aufrechterhaltung einer Temperatur von 0 bis 50 ^{o}C in der Suspension,
dadurch gekennzeichnet,
daß die Sprühdüse auch in der Anfahr- und Abstellungsphase stets in die wäßrige Flüssigkeit eingetaucht ist und man unmittelbar vor und nach dem Versprühen durch die mit geschmolzenem Cyanurchlorid in Berührung kommenden Teile der Sprühvorrichtung überhitzten Wasserdampf leitet.

2. Verfahren nach Anspruch 1,
dadurch gekennzeichnet,
daß man geschmolzenes Cyanurchlorid in Form eines Gemischs aus geschmolzenem Cyanurchlorid und überhitztem Wasserdampf versprüht.

3. Verfahren nach Anspruch 2,
dadurch gekennzeichnet,
daß man das Gemisch unter Verwendung einer Zweistoffdüse mit innenliegender Mischzone herstellt und unmittelbar versprüht.

4. Verfahren nach Anspruch 2 oder 3,
dadurch gekennzeichnet,
daß man überhitzten Wasserdampf und geschmolzenes Cyanurchlorid im Gewichtsverhältnis von 1 zu 15 bis 1 zu 25 mischt.

5. Verfahren nach Anspruch 1,
dadurch gekennzeichnet,
daß man geschmolzenes Cyanurchlorid in Form eines mit der genannten wäßrigen Flüssigkeit im wesentlichen umhüllten Schmelzstrahls versprüht.

6. Verfahren nach Anspruch 5,
dadurch gekennzeichnet,
daß man eine Sprühvorrichtung mit einer Zweistoffdüse verwendet, wobei dem in der Düse zentral geführten Cyanurchlorid-Schmelzstrahl mittels eines am Düsenmund angebrachten Ringspalts die den Schmelzstrahl umhüllende wäßrige Flüssigkeit zugeführt wird.

7. Verfahren nach einem oder mehreren der Ansprüche 1 bis 6,
dadurch gekennzeichnet,
daß man die Düsenöffnung der Sprühvorrichtung unmittelbar vor der Saugseite einer Maschine zur Naßzerkleinerung anordnet.

## Claims

1. A process for the production of a suspension of cyanuric chloride in an aqueous liquid, more particularly in water, the aqueous liquid optionally containing - in addition to water - dissolved organic solvents and/or reactants for the further reaction of the suspended cyanuric chloride formed, by spraying molten cyanuric chloride into the above-mentioned aqueous liquid, optionally already containing suspended cyanuric chloride, using a spray arrangement comprising a spray nozzle dipping into the aqueous liquid and maintaining a temperature of 0 to 50°C in the suspension, characterized in that the spray nozzle is always immersed in the aqueous liquid, even during the start and stop phases, and superheated steam is passed through those parts of the spray arrangement which come into contact with molten cyanuric chloride immediately before and after the spraying process.

2. A process as claimed in claim 1, characterized in that molten cyanuric chloride is sprayed in the form of a mixture of molten cyanuric chloride and superheated steam.

3. A process as claimed in claim 2, characterized in that the mixture is prepared and immediately sprayed in a two-component nozzle with an internal mixing zone.

4. A process as claimed in claim 2 or 3, characterized in that superheated steam and molten cyanuric chloride are mixed in a ratio by weight of 1:15 to 1:25.

5. A process as claimed in claim 1, characterized in that molten cyanuric chloride is sprayed in the form of a melt stream substantially surrounded by the aqueous liquid mentioned.

6. A process as claimed in claim 5, characterized in that a spray arrangement comprising a two-component nozzle is used, the aqueous liquid surrounding the cyanuric chloride melt stream guided centrally in the nozzle being delivered thereto by means of an annular gap situated at the nozzle orifice.

7. A process as claimed in one or more of claims 1 to 6, characterized in that the nozzle orifice of the spray arrangement is arranged immediately in front of the suction side of a wet size-reducing machine.

## Revendications

1. Procédé d'obtention d'une suspension de chlorure de cyanuryle dans un liquide aqueux, en particulier dans l'eau, dans lequel le liquide aqueux autre que l'eau peut renfermer des solvants organiques dissous dans celle-ci et/ou des partenaires de réaction en vue d'une réaction ultérieure du chlorure de cyanuryle en suspension, formé, par pulvérisation de chlorure de cyanuryle fondu dans le liquide aqueux mentionné renfermant du chlorure de cyanuryle le cas échéant déjà en suspension, en utilisant un dispositif de pulvérisation avec une buse de pulvérisation immergée dans le liquide aqueux et en maintenant une température de 0 à 50°C dans la suspension, caractérisé en ce que la buse de pulvérisation est immergée aussi pendant les phases de démarrage et d'arrêt en permanence dans le liquide aqueux et en ce que l'on conduit immédiatement avant et après la pulvérisation de la vapeur d'eau surchauffée à travers les parties du dispositif de pulvérisation qui viennent en contact avec le chlorure de cyanuryle fondu.

2. Procédé selon la revendication 1, caractérisé en ce que l'on pulvérise du chlorure de cyanuryle fondu sous forme d'un mélange à base de chlorure de cyanuryle fondu et de vapeur d'eau surchauffée.

3. Procédé selon la revendication 2, caractérisé en ce que l'on produit le mélange en utilisant une buse pour deux postes ayant une zone de mélange située intérieurement et que l'on pulvérise immédiatement.

4. Procédé selon la revendication 2 ou 3, caractérisé en ce que l'on mélange la vapeur d'eau surchauffée et le chlorure de cyanuryle fondu dans un rapport pondéral de 1 pour 15 à 1 pour 25.

5. Procédé selon la revendication 1, caractérisé en ce que l'on pulvérise le chlorure de cyanuryle sous forme d'un jet de produit de fusion enrobé pour l'essentiel avec le liquide aqueux mentionné.

6. Procédé selon la revendication 5, caractérisé en ce que l'on utilise un dispositif de pulvérisation ayant une buse pour deux postes dans lequel on amène au jet de produit de fusion de chlorure de cyanuryle conduit d'une manière centrale dans la buse, le liquide aqueux qui enrobe le jet de produit de fusion a l'aide d'une fente annulaire appliquée sur l'embouchure de la buse.

7. Procédé selon l'une ou plusieurs des revendications 1 à 6, caractérisé en ce que l'on dispose l'ouverture de la buse du dispositif de pulvérisation directement devant le côté d'aspiration d'une machine pour broyage humide.
